(19) Europäisches Patentamt
European Patent Office
Office européen des brevets

(11) Veröffentlichungsnummer : **0 018 531**
**B1**

(12) # EUROPÄISCHE PATENTSCHRIFT

(45) Veröffentlichungstag der Patentschrift : **26.01.83**

(51) Int. Cl.³ : **C 07 C 69/734**, C 07 C 69/757

(21) Anmeldenummer : **80101997.7**

(22) Anmeldetag : **14.04.80**

(54) **Substituierte 2,2-Dimethylpentan (en)-1-carbonsäurederivate, Verfahren zu ihrer Herstellung und ihre Verwendung zur Herstellung von Styrylcyclopropancarbonsäuren.**

(30) Priorität : **23.04.79 DE 2916375**

(43) Veröffentlichungstag der Anmeldung : **12.11.80 Patentblatt 80/23**

(45) Bekanntmachung des Hinweises auf die Patenterteilung : **26.01.83 Patentblatt 83/04**

(84) Benannte Vertragsstaaten : **BE CH DE FR GB IT LI NL**

(56) Entgegenhaltungen :
**DE A 2 539 895**
**DE A 2 706 184**
**DE A 2 738 150**
**DE A 2 740 849**
**DE A 2 750 182**
**DE A 2 800 073**
**DE A 2 827 101**
**DE A 2 848 495**
**US A 4 157 447**

(73) Patentinhaber : **BAYER AG**
**Zentralbereich Patente, Marken und Lizenzen**
**D-5090 Leverkusen 1, Bayerwerk (DE)**

(72) Erfinder : **Jautelat, Manfred, Dr.**
**Müllersbaum 28**
**D-5093 Burscheid 1 (DE)**
Erfinder : **Arlt, Dieter, Dr.**
**Rybniker Strasse 2**
**D-5000 Köln 80 (DE)**
Erfinder : **Lantzsch, Reinhard, Dr.**
**Heymannstrasse 32**
**D-5090 Leverkusen (DE)**
Erfinder : **Fuchs, Rainer, Dr.**
**Roeberstrasse 8**
**D-5600 Wuppertal 1 (DE)**
Erfinder : **Riebel, Hans-Jochem, Dr.**
**In der Beek 92**
**D-5600 Wuppertal 1 (DE)**
Erfinder : **Schröder, Rolf, Dr.**
**Pahlkestrasse 17**
**D-5600 Wuppertal 1 (DE)**
Erfinder : **Harnisch, Horst, Dr.**
**Heinenbusch 4**
**D-5203 Much (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 018 531 B1

# Substituierte 2,2-Dimethylpentan (en)-1-carbonsäurederivate, Verfahren zu ihrer Herstellung und ihre Verwendung zur Herstellung von Styrylcyclopropancarbonsäuren

Die vorliegende Erfindung betrifft substituierte 2,2-Dimethylpentan (en)-1-carbonsäurederivate, Verfahren zu ihrer Herstellung und ihre Verwendung zur Herstellung von Styryl-cyclopropan-carbonsäureestern.

Es ist bereits bekannt geworden, daß Ester von 3-Styryl-2,2-dimethylcyclopropancarbonsäuren insektizide Eigenschaften besitzen (Deutsche Offenlegungsschrift 2 738 150). Sie werden hergestellt, indem die Knüpfung der C-C-Doppelbindung der Styrylgruppe durch eine Wittigreaktion, bei der Butyllithium als Base verwendet wird und unter Schutzgas bei – 78 °C gearbeitet werden muß, erfolgt. Dieser Syntheseweg ist für eine technische Herstellung nicht gangbar.

Ferner ist der als Ausgangsprodukt für diese Reaktion verwendete 2,2-Dimethyl-3-formyl-1-carbonsäureester technisch nur schwer zugänglich.

Gegenstand der vorliegenden Erfindung sind :

1. Substituierte 2,2-Dimethylpentan (en)-1-carbonsäurederivate der Formel

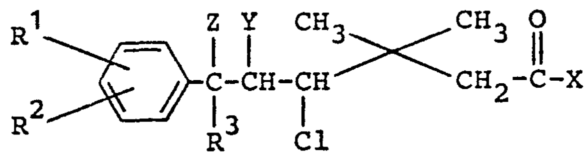

in welcher

X für Cl oder —O—R steht,

R für $C_{1-4}$-Alkyl oder für den Rest eines gegebenenfalls substituierten Phenoxybenzylalkohols der Formel

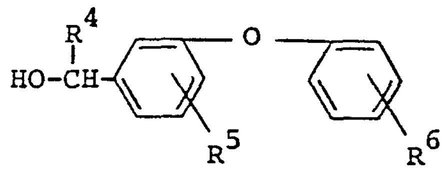

in welcher

$R^4$ für Wasserstoff, CN, —C ≡ CH steht,

$R^5$ und $R^6$ für Wasserstoff oder Fluor stehen,

Y für Wasserstoff steht

Z für Chlor oder gemeinsam mit Y für eine Doppelbindung steht,

$R^1$ für Alkoxy oder Alkylthio, die gegebenenfalls durch Halogen substituiert sind, steht,

$R^2$ für Wasserstoff, Alkoxy oder gemeinsam mit $R^1$ für gegebenenfalls halogensubstituiertes Alkylendioxy steht,

$R^3$ für Wasserstoff oder Chlor steht.

2. Verfahren zur Herstellung von Verbindungen der Formel

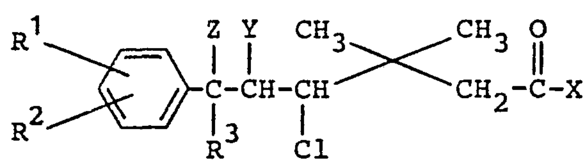

in welcher

X für Cl oder —O—R steht,

R für $C_{1-4}$-Alkyl oder für den Rest eines gegebenenfalls substituierten Phenoxybenzylalkohols der Formel

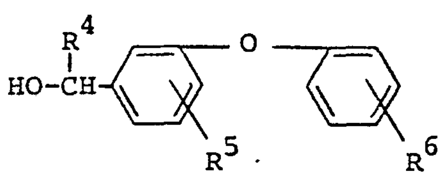

in welcher

$R^4$ für Wasserstoff, CN, —C ≡ CH steht,
$R^5$ und $R^6$ für Wasserstoff oder Fluor stehen,
Y für Wasserstoff steht
Z für Chlor oder gemeinsam mit Y für eine Doppelbindung steht,
$R^1$ für Alkoxy oder Alkylthio, die gegebenenfalls durch Halogen substituiert sind, steht,
$R^2$ für Wasserstoff, Alkoxy oder gemeinsam mit $R^1$ für gegebenenfalls halogensubstituiertes Alkylendioxy steht,
$R^3$ für Wasserstoff oder Chlor steht,
dadurch gekennzeichnet, daß man

a) für den Fall, daß Y und Z gemeinsam für eine Doppelbindung stehen,

$a_1$) aus Verbindungen der obigen Formel, in welcher
Z und $R^3$ für Chlor stehen und Y für Wasserstoff steht, thermisch Chlorwasserstoff abspaltet, oder

$a_2$) Verbindungen der obigen Formel, in welcher
Z und $R^3$ für Chlor stehen und
Y für Wasserstoff steht und
X für Chlor steht, mit Alkoholen der Formel

$$R—O—H$$

in welcher
R die oben angegebene Bedeutung besitzt, umsetzt, oder

$a_3$) Verbindungen der Formel VI

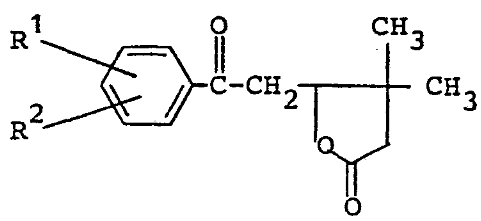

(VI)

in welcher
$R^1$ und $R^2$ die oben angegebene Bedeutung haben, mit mindestens zwei Äquivalenten Phosphorpentachlorid umsetzt und anschließend mit einem Alkohol der Formel

$$R—O—H$$

in welcher
R die oben angegebene Bedeutung besitzt, umsetzt oder

$a_4$) Verbindungen der Formel (VI) (oben) reduziert, Wasser abspaltet und mit einem Chlorierungsmittel in Gegenwart eines Alkohols der Formel

$$R—OH$$

in welcher
R die oben angegebene Bedeutung besitzt, umsetzt oder aber nacheinander zunächst den Lactonring mit einem Chlorierungsmittel öffnet und anschließend mit dem Alkohol umsetzt oder

b) für den Fall, daß Z und $R^3$ für Cl stehen und Y für H steht, Verbindungen der Formel (V)

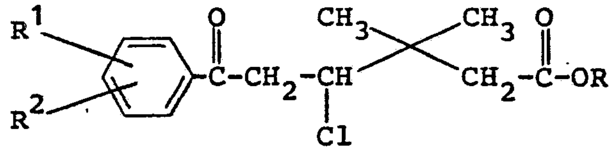

(V)

in welcher
R, $R^1$, $R^2$ die oben angegebene Bedeutung besitzen, mit Phosphorpentachlorid in einem Verdünnungsmittel unterhalb 30 °C umsetzt, oder

c) für den Fall, daß Z und $R^3$ für Cl stehen und Y für H steht und X für Cl steht, Verbindungen der Formel (VI) (oben)

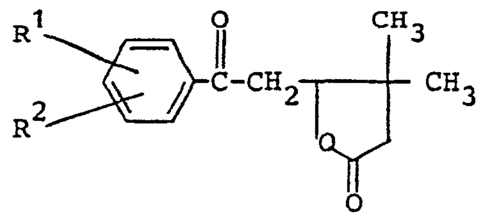

(VI)

in welcher

$R^1$ und $R^2$ die oben angegebene Bedeutung haben, mit mindestens zwei Äquivalenten Phosphorpentachlorid in einem Verdünnungsmittel unterhalb 30 °C umsetzt.

3. Verwendung von Verbindungen der Formel

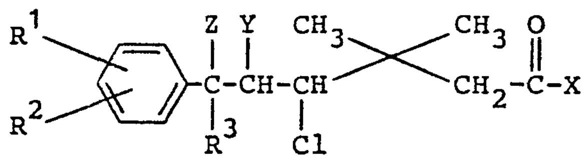

in welcher

X für Cl oder —O—R steht,

R für $C_{1-4}$-Alkyl oder für den Rest eines gegebenenfalls substituierten Phenoxybenzylalkohols der Formel

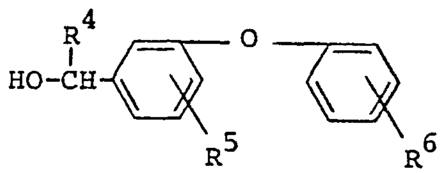

in welcher

$R^4$ für Wasserstoff, CN, —C ≡ CH steht,

$R^5$ und $R^6$ Wasserstoff oder Fluor stehen,

Y für Wasserstoff steht

Z für Chlor oder gemeinsam mit Y für eine Doppelbindung steht,

$R^1$ für Alkoxy oder Alkylthio, die gegebenenfalls durch Halogen substituiert sind, steht,

$R^2$ für Wasserstoff, Alkoxy oder gemeinsam mit $R^1$ für gegebenenfalls halogensubstituiertes Alkylendioxy steht,

$R^3$ für Wasserstoff oder Chlor steht, zur Herstellung von Styryl-cyclopropancarbonsäureestern der allgemeinen Formel (I)

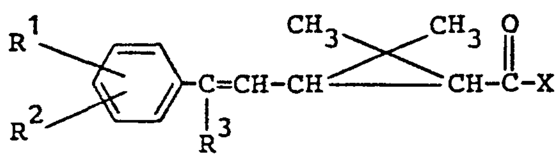

(I)

in der X, $R^1$, $R^2$, $R^3$ die oben angegebene Bedeutung haben, dadurch gekennzeichnet, daß man Verbindungen der Formel

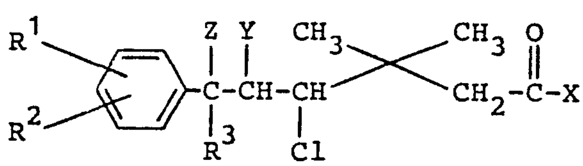

in welcher

$R^1$, $R^2$, $R^3$, X, Y, Z die oben angegebene Bedeutung haben, mit einer äquivalenten Menge einer Base

gegebenenfalls in Gegenwart eines Verdünnungsmittels zwischen − 20 und 60 °C umsetzt, und gegebenenfalls mit einem Alkohol der Formel R—O—H weiter umsetzt.

Verbindungen der Formel (II)

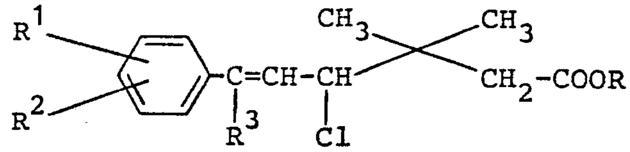

(II)

in welcher

R-$R^3$ die oben angegebene Bedeutung haben.

Verbindungen der Formel (III)

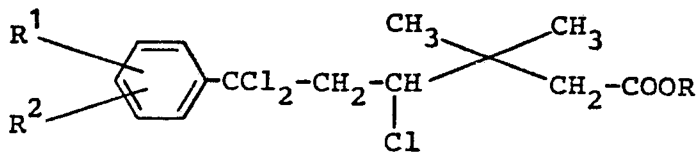

(III)

in welcher

R-$R^3$ die oben angegebene Bedeutung haben.

Verbindungen der Formel (IV)

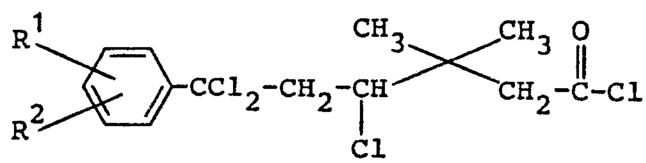

(IV)

in welcher

$R^1$ und $R^2$ die oben angegebene Bedeutung haben.

4. Verbindungen der allgemeinen Formel (V) werden erhalten, indem man Verbindungen der Formel (VI) mit einem Chlorierungsmittel in Gegenwart eines Alkohols R—OH umsetzt, wobei R die unter 1. (oben) angegebene Bedeutung besitzt oder aber nacheinander zunächst den Lactonring mit einem Chlorierungsmittel öffnet und anschliessend mit dem Alkohol umsetzt.

5. Verbindungen der allgemeinen Formel (VI) werden erhalten indem man Verbindungen der Formel (VII)

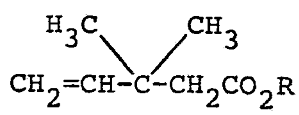

(VII)

in der

R $C_1$-$C_4$-Alkyl bedeutet, mit Verbindungen der Formel (VIII)

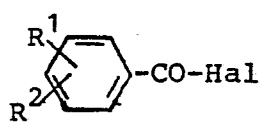

(VIII)

in welcher

$R^1$ und $R^2$ die unter 1. (oben) angegebene Bedeutung besitzen und

Hal Halogen, vorzugsweise Chlor bedeutet, in Gegenwart eines Friedel-Crafts-Katalysators umsetzt.

6. Verbindungen der allgemeinen Formel (VIa)

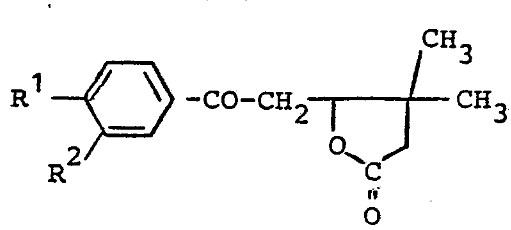

(VIa)

in welcher

$R^1$ für Alkoxy oder Alkylthio steht

$R^2$ für Wasserstoff, Alkoxy oder gemeinsam mit $R^1$ für Alkylendioxy steht, werden erhalten, indem man Verbindungen der allgemeinen Formel (X)

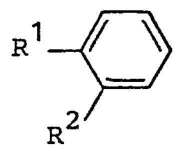

(X)

in welcher

$R^1$ und $R^2$ die oben angegebene Bedeutung besitzen, mit dem Säurechlorid der Formel (XI)

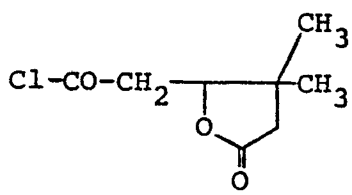

(XI)

in Gegenwart eines Friedel-Crafts-Katalysators sowie gegebenenfalls in Gegenwart eines Verdünnungsmittels umsetzt.

7. Die Verbindung der Formel (XI) wird hergestellt, indem man die Verbindung der Formel (XII)

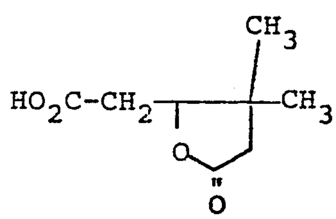

(XII)

mit einem Chlorierungsmittel umsetzt.

Die nach dem erfindungsgemäßen Verfahren 3 (oben) herstellbaren Styrylcyclopropancarbonsäuren der Formel I sind insektizid und akarizid wirksam.

Vorzugsweise werden gemäß Verfahren 3 (oben) Styrylcyclopropancarbonsäurederivate der Formel (I) erhalten, in welcher

$R^1$ für $C_1$-$C_4$-Alkoxy, $C_1$-$C_4$-Alkylthio, $C_1$-$C_2$-Fluoralkoxy, $C_1$-$C_2$-Chlorfluoralkoxy oder $C_1$-$C_2$-Fluoralkylthio steht,

$R^2$ für Wasserstoff, Methoxy oder zusammen mit $R^1$ für $C_1$-$C_2$-Alkylendioxy oder $C_1$-$C_2$-Fluoralkylendioxy steht,

$R^3$ für Chlor steht,

R für $C_{1-4}$-Alkyl oder für den Rest eines gegebenenfalls substituierten Phenoxybenzylalkohols der allgemeinen Formel (III)

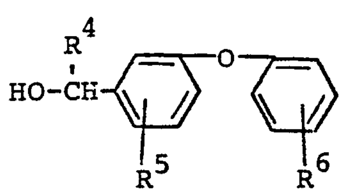

(III)

steht, worin

$R^4$ für Wasserstoff, Cyano oder Äthinyl steht und

$R^5$ und $R^6$ für Wasserstoff oder Fluor stehen.

Verwendet man 6-(4'-Methoxy-phenyl)-4,6-dichlor-3,3-dimethyl-hex-5-en-säureäthylester als Ausgangsstoff, kann der Reaktionsablauf durch folgendes Formelschema wiedergegeben werden :

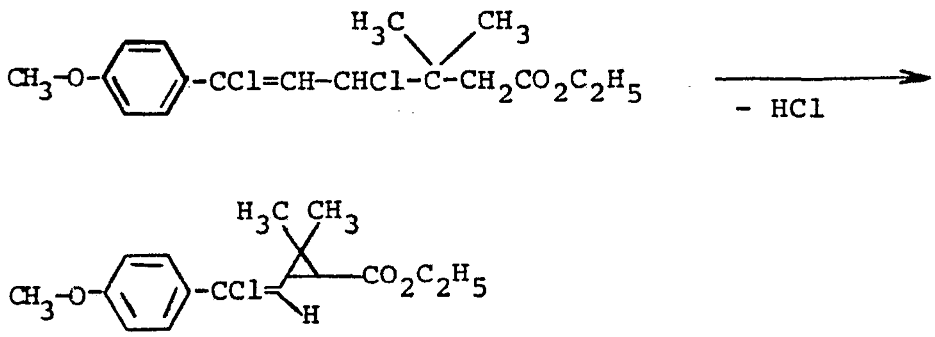


Verwendet man 6-(4'-Methoxy-phenyl)-4,6,6-trichlor-3,3-dimethyl-hexansäuremethylester als Ausgangsstoff, kann der Reaktionsablauf durch folgendes Formelschema wiedergegeben werden :

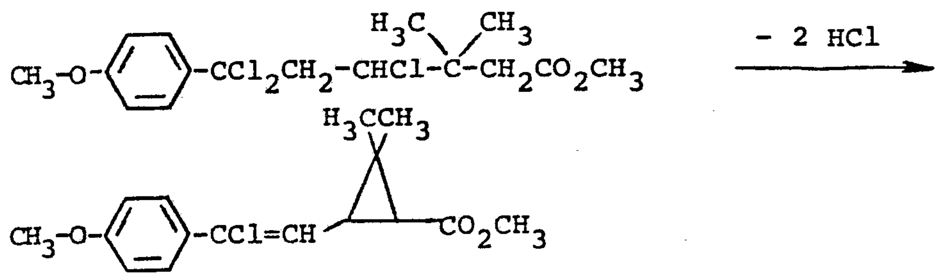


Verwendet man 6-(4'-Methoxy-phenyl)-4,6,6-trichlor-3,3-dimethyl-hexansäurechlorid als Ausgangsstoff, kann der Reaktionsverlauf durch folgendes Formelschema wiedergegeben werden :

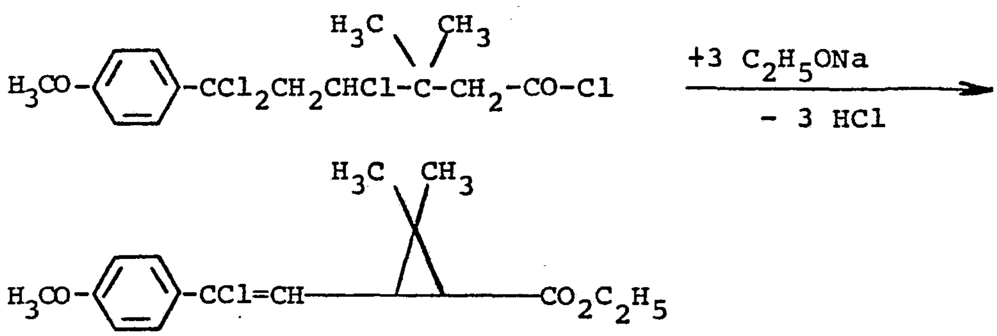


Verfahren 3 wird durchgeführt, indem man den Ausgangsstoff mit 1, 2 bzw. 3 Äquivalenten Base gegebenenfalls in Gegenwart eines Verdünnungsmittels umsetzt.

Als Basen finden bevorzugt Alkoholate Verwendung, wie Natriummethylat, Kaliumäthylat, Natriumäthylat, Natriumisopropylat, Natrium-tert.-butylat oder Kalium-tert.-butylat.

Als Verdünnungsmittel wird bevorzugt der der Base entsprechende Alkohol eingesetzt, es können jedoch auch zusätzlich oder ausschließlich andere inerte Verdünnungsmittel verwendet werden, wie beispielsweise Kohlenwasserstoffe, wie Toluol, Xylol, Cyclohexan oder Chlorkohlenwasserstoffe, wie Chlorbenzol oder Äther, wie Diisopropyläther, THF oder Dioxan.

Das Verfahren wird bei Temperaturen zwischen – 20 °C und + 60 °C, vorzugsweise zwischen 20 °C und 50 °C durchgeführt.

Eine ähnliche Reaktion ist in der DE-OS 2 539 896 beschrieben. Doch während dort der bevorzugte Temperaturbereich — bei Anwendung von Natriummethylat oder Natriumäthylat — zwischen 60 und 100 °C liegt (Seite 31), wird unter diesen Bedingungen nur sehr wenig Produkt der Formel (I) erhalten, sondern hauptsächlich ein weiteres Mol Halogenwasserstoff unter Bildung einer Dreifachbindung abgespalten.

Überraschend wurde gefunden, daß dies vermieden wird, wenn unterhalb von 60 °C gearbeitet wird.

Ein weiterer Vorteil des erfindungsgemäßen Verfahrens ist, daß ganz bevorzugt nur eines der 4 möglichen Stereoisomeren gebildet wird. Es besitzt die trans-Figuration bezogen auf den Cyclopropanring.

Die insektiziden und akariziden Ester dieses Isomeren zeigen eine besonders gute Wirksamkeit.

Nach Verfahren 3. lassen sich bevorzugt folgende Cyclopropancarbonsäureester der Formel (I) herstellen.

2,2-Dimethyl-3-[2'-chlor-2'-(4'-trifluormethylmercapto-phenyl)-vinyl]-cyclopropan-1-carbonsäure-methyl und -äthylester

2,2-Dimethyl-3-[2'-chlor-2'-(3",4"-dimethoxy-phenyl)-vinyl]-cyclopropan-1-carbonsäuremethyl und -äthylester

2,2-Dimethyl-3-[2'-chlor-2'-(4'-brom-phenyl)-vinyl]-cyclopropan-1-carbonsäuremethyl- und äthylester

2,2-Dimethyl-3-[2'-chlor-2'-(3,4-trifluoräthylendioxy-phenyl)-vinyl]-cyclopropan-1-carbonsäuremethyl- und äthylester

2,2-Dimethyl-3-[2'-chlor-2'-(3'-trifluormethylmercapto-phenyl)-vinyl]-cyclopropan-1-carbonsäuremethyl- und äthylester

2,2-Dimethyl-3-[2'-chlor-2'-(4'-methylmercapto-phenyl)-vinyl]-cyclopropan-1-carbonsäuremethyl- und äthylester

2,2-Dimethyl-3-[2'-chlor-2'-(4'-äthoxy-phenyl)-vinyl]-cyclopropan-1-carbonsäuremethyl- und äthylester

2,2-Dimethyl-3-[2'-chlor-2'-(4'-methoxy-phenyl)-vinyl]-cyclopropan-1-carbonsäuremethyl- und äthylester

2,2-Dimethyl-3-[2'-chlor-2'-(4'-trifluormethoxy-phenyl)-vinyl]-cyclopropan-1-carbonsäuremethyl- und äthylester

2,2-Dimethyl-3-[2'-chlor-2'-(3'-trifluormethoxy-4'-chlor-phenyl)-vinyl]-cyclopropan-1-carbonsäuremethyl- und äthylester

2,2-Dimethyl-3-(2'-chlor-2'-(3',4'-methylendioxy-phenyl)-vinyl]-cyclopropan-1-carbonsäuremethyl- und äthylester.

Die Verbindungen der allgemeinen Formel (II) gemäß 3 (oben) sind neu. Sie werden nach dem unter 2a (oben) angegebenen Verfahren erhalten, indem man $a_1$) aus Verbindungen der allgemeinen Formel (III) thermisch Chlorwasserstoff abspaltet oder $a_2$) Verbindungen der Formel (IV) mit Alkoholen umsetzt oder $a_3$) Verbindungen der Formel (VI) mit Phosphorpentachlorid und anschließend mit einem Alkohol umsetzt oder $a_4$) Verbindungen der Formel VI reduziert, Wasser abspaltet und mit einem Chlorierungsmittel in Gegenwart eines Alkohols umsetzt oder nacheinander zunächst den Lactonring mit einem Chlorierungsmittel öffnet und anschließend mit einem Alkohol umsetzt.

Verwendet man bei Verfahren $2a_1$ 6-(4'-Methoxy-phenyl)-4,6,6-trichlor-3,3-dimethyl-hexansäureäthylester als Ausgangsstoff, kann der Reaktionsablauf durch folgendes Formelschema wiedergegeben werden :

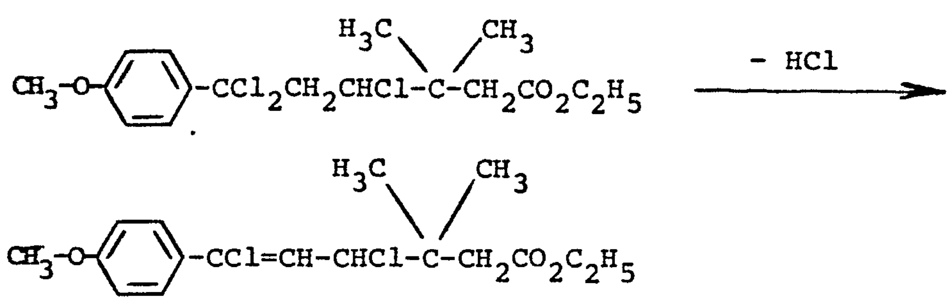


Die beim Verfahren $2a_1$ verwendbaren Ausgangsstoffe sind durch die allgemeine Formel (II) definiert. Die bevorzugten Substituenten $R^1$, $R^2$ und R sind die gleichen wie bei Verfahren 3.

Die Verbindungen der Formel (III) sind neu ; ihre Herstellung wird weiter unten beschrieben.

Verfahren $2a_1$ wird durchgeführt, indem man die Ausgangsstoffe der Formel (III), gegebenenfalls in einem Verdünnungsmittel, auf Temperaturen zwischen 25 und 80 °C, vorzugsweise auf Temperaturen zwischen 30 und 50 °C, erwärmt. Hierbei spaltet sich Chlorwasserstoff ab. Als Verdünnungsmittel kommen insbesondere Kohlenwasserstoffe wie Benzol, Toluol, Xylol, Benzin, Cyclohexan, Petroläther in Frage, aber auch Chlorkohlenwasserstoffe wie Methylenchlorid, Chloroform, Tetrachlorkohlenstoff, Dichloräthan oder Chlorbenzol, sowie Nitrile, wie Acetonitril, in Frage.

Gegebenenfalls kann auf die Isolierung der Verbindungen der allgemeinen Formel (III) verzichtet werden, so daß sich Verfahren, $2a_1$ unmittelbar an Verfahren 2b (unten) anschließt.

Verwendet man bei Verfahren $2a_2$ 6-(4'-Methoxy-phenyl)-4,6,6-trichlor-3,3-dimethylhexansäurechlorid als Ausgangsstoff, kann der Reaktionsverlauf durch folgendes Formelschema wiedergegeben werden :

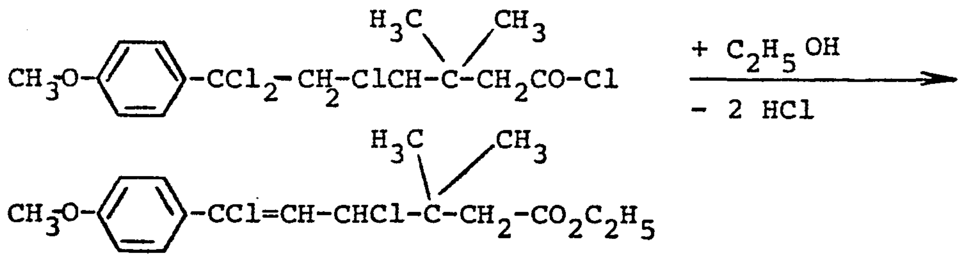

Die beim Verfahren $2a_2$ verwendbaren Ausgangsstoffe sind durch die allgemeine Formel (IV) definiert. Die bevorzugten Substituenten $R^1$ und $R^2$ sind die gleichen wie bei Verfahren 3.

Die Verbindungen der Formel (IV) sind neu ; ihre Herstellung wird weiter unten beschrieben.

Verfahren $2a_2$ wird durchgeführt, indem man die Ausgangsstoffe der Formel (IV) gegebenenfalls in einem Verdünnungsmittel bei Temperaturen zwischen – 20 °C und 80 °C, vorzugsweise zwischen 0 °C und 30 °C mit einem Alkohol der allgemeinen Formel R—OH versetzt, wobei die bevorzugte Bedeutung von R die gleiche ist, wie bei Verfahren 3. Zur Beendigung der Reaktion wird anschließend noch eine Zeitlang bei erhöhter Temperatur, vorzugsweise zwischen 30 und 60 °C gerührt. Als Verdünnungsmittel kann Überschüssiger Alkohol verwendet werden, sowie alle Lösungsmittel, die auch in Verfahren $2a_1$ Verwendung finden können.

Verwendet man bei Verfahren $2a_3$ 3,3-Dimethyl-4-(4'-methoxy-phenacyl)-γ-butyrolacton und Äthanol als Ausgangsstoffe, kann der Reaktionsablauf durch folgendes Formelschema wiedergegeben werden :

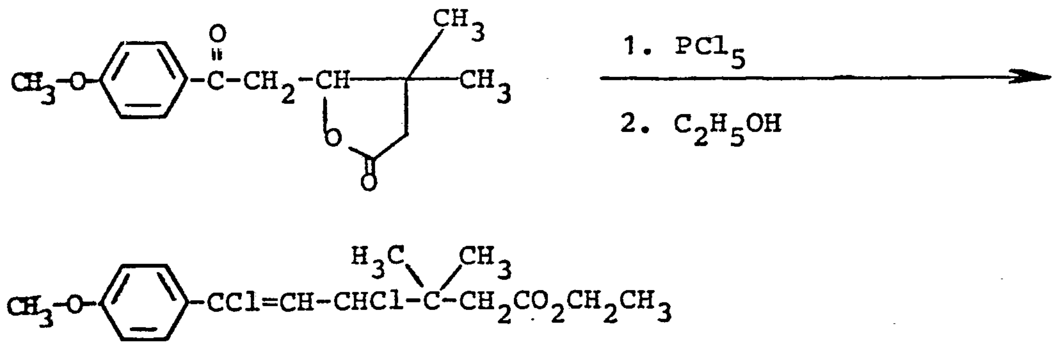


Die beim Verfahren $2a_3$ verwendbaren Ausgangsstoffe sind durch die allgemeine Formel (VI) definiert. Die bevorzugten Substituenten $R^1$ und $R^2$ sind die gleichen wie bei Verfahren 3. Die Verbindungen der Formel (VI) sind neu ; ihre Herstellung wird weiter unten beschrieben.

Als Chlorierungsmittel kann Phosphorpentachlorid verwendet werden.

Verfahren $2a_3$ wird in der Weise durchgeführt, daß man die Ausgangsstoffe der allgemeinen Formel (V) vorlegt und mit mindestens zwei Äquivalenten des Chlorierungsmittels umsetzt. Ein geringer Überschuß ist günstig.

Verfahren $2a_3$ wird in der Weise durchgeführt, daß man die Ausgangsstoffe der allgemeinen Formel (VI) mit mindestens zwei Äquivalenten des Chlorierungsmittels umsetzt. Ein geringer Überschuß ist günstig.

Vorzugsweise wird — im Gegensatz zur allgemein üblichen Arbeitsweise (Houben-Weyl, Band V, 3, Seite 912 ff) bei der Umsetzung von Ketonen mit Phosphorpentachlorid — in Gegenwart eines Verdünnungsmittels gearbeitet. Als Verdünnungsmittel kommen die gleichen wie die bei Verfahren $2a_1$ genannten in Frage.

Durch Anwendung eines Verdünnungsmittels sowie Zudosieren des Phosphorpentachlorids können überraschenderweise die sonst üblichen Nebenreaktionen, wie beispielsweise eine α-Chlorierung zur Carbonylgruppe oder eine Addition von Chlorwasserstoff an die Chlorvinylgruppe vermieden werden.

Die Umsetzungstemperatur liegt zwischen – 20 °C und + 60 °C, vorzugsweise zwischen 0 °C und 35 °C.

Die anschließende Veresterung erfolgt durch Zutropfen von überschüssigem Alkohol R—OH analog Verfahren $2a_2$.

Die Aufarbeitung erfolgt durch Neutralwaschen und Abtrennen der organischen Phase, sowie Abdestillieren des Lösungsmittels un des Phosphorsäureesters. Auf eine Reinigung durch Destillation kann auf dieser Stufe meist verzichtet werden.

Verwendet man bei Verfahren $2a_4$ 3,3-Dimethyl-4-(methoxy-phenacyl)-γ-butylrolacton als Ausgangsstoff, kann der Reaktionsverlauf durch folgendes Formelschema wiedergegeben werden :

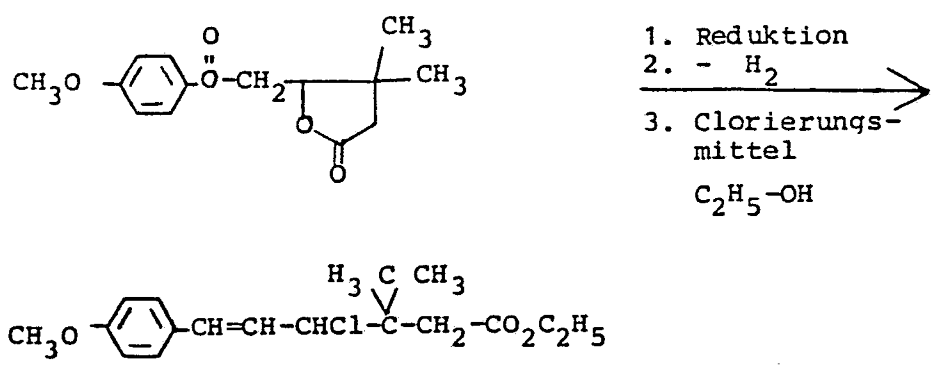

Die beim Verfahren $2a_4$ verwendbaren Ausgangsstoffe sind durch die allgemeine Formel (VI) definiert. Die bevorzugten und besonders bevorzugten Substituenten Ar sind die gleichen wie bei Verfahren 3. Die Verbindungen der Formel (VI) sind neu ; ihre Herstellung wird weiter unten beschrieben.

Als Reduktionsmittel kommen grundsätzlich alle Mittel in Frage, durch die ein Keton zum Alkohol reduziert wird, ohne den Lactonring anzugreifen. Beispielsweise seien genannt : komplexe Borhydride, wie Natriumborhydrid oder Wasserstoff in Gegenwart von beispielsweise Nickel-, Palladium- oder Platin-Katalysatoren, wie beispielsweise Raney-Nickel. Natriumborhydrid ist bevorzugt.

Verfahren $2a_4$ wird in der Weise durchgeführt, daß man nach dem Reduktionsschritt, der in an sich üblicher Weise durchgeführt wird, dehydratisiert, d.h. Wasser abspaltet.

Zur Dehydratisierung werden bevorzugt saure Katalysatoren-eingesetzt. Beispielsweise seien genannt : Oxalsäure, Schwefelsäure, Phosphorsäure, Kaliumhydrogensulfat, p-Toluolsulfonsäure, Aluminium oxid, Silikate. Außerdem kann der entstandene Alkohol-acetylrest und anschließend durch Erhitzen Essigsäure abgespalten werden. Die Acetylierung erfolgt mit Acetylchlorid oder Acetanhydrid. Der dritte Teilschritt des Verfahrens $2a_4$ entspricht Verfahren 4 (unten).

Verwendet man bei Verfahren 2b 6-(3'-Methoxy-phenyl)-6-oxo-4-chlor-3,3-dimethyl-hexansäureäthylester als Ausgangsstoff, so kann der Reaktionsablauf durch folgendes Formelschema wiedergegeben werden :

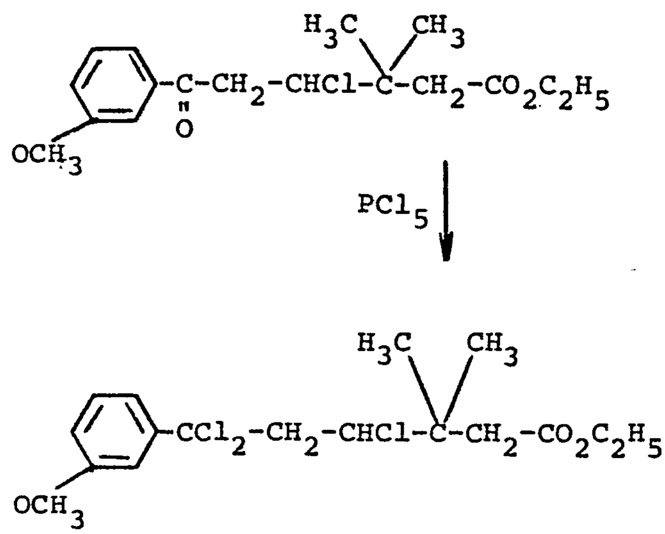


Die beim Verfahren 2b verwendbaren Ausgangsstoffe sind durch die allgemeine Formel (V) definiert. Die bevorzugten Substituenten $R^1$, $R^2$ und R sind die gleichen wie bei Verfahren 3. Die Verbindungen der Formel (V) sind neu ; ihre Herstellung wird weiter unten beschrieben.

Es wurde gefunden, daß nur dann die Verbindungen (III) aus den Verbindungen der Formel (V) erhalten werden, wenn man in einem Verdünnungsmittel unterhalb von 30 °C arbeitet.

Verfahren 2b wird in der Weise durchgeführt, daß man die Ausgangsstoffe der allgemeinen Formel (III) in einem Verdünnungsmittel löst. Als Verdünnungsmittel kommen die gleichen wie die bei Verfahren 6a genannten in Frage, bevorzugt sind Petroläther, Cyclohexan, Toluol und Chlorbenzol.

Die Umsetzungstemperatur liegt zwischen − 20 °C und 30 °C, vorzugsweise zwischen 0 und 25 °C.

Die Aufarbeitung erfolgt durch Versetzen mit Eiswasser, neutral waschen der organischen Phase und Abdestillieren des Lösungsmittels.

Der Konstitutionsbeweis erfolgt durch ein Kernresonanzspektrum.

Verwendet man bei Verfahren 2c, 3,3-Dimethyl-4-(4'-methoxy-phenacyl)-γ-butyrolacton als Ausgangsstoff, kann der Reaktionsverlauf durch folgendes Formelschema wiedergegeben werden :

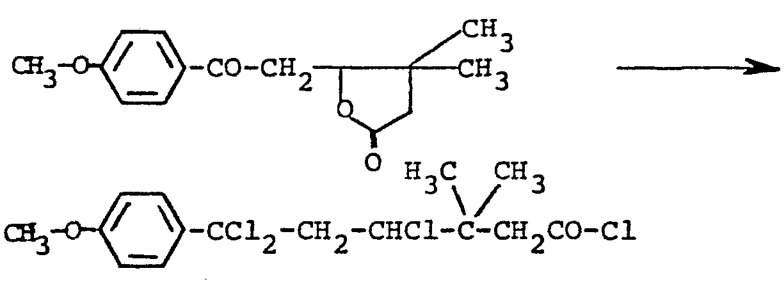

Die beim Verfahren 2c verwendbaren Ausgangsstoffe sind durch die allgemeine Formel (VI) definiert. Die bevorzugten Substituenten sind die gleichen wie bei Verfahren 3. Die Verbindungen der Formel (VI) sind neu ; ihre Herstellung wird weiter unten beschrieben.

Es wurde weiter gefunden, daß nur dann die Verbindungen (IV) aus den Verbindungen (VI) erhalten werden, wenn man in einem Verdünnungsmittel unterhalb von 30 °C arbeitet.

Verfahren 2c wird in der Weise durchgeführt, daß man die Ausgangsstoffe der allgemeinen Formel (VI) in einem Verdünnungsmittel löst.

Als Verdünnungsmittel kommen die gleichen wie die bei Verfahren $2a_1$ genannten in Frage, bevorzugt sind Petroläther, Cyclohexan, Toluol und Chlorbenzol.

Die Umsetzungstemperatur liegt zwischen − 20 ° und + 30 °C, vorzugsweise zwischen 0 und 25 °C.

Die Verbindungen der Formel (IV) können durch schonendes Abdestillieren von Lösungsmittel und Phosphoroxychorid isoliert werden oder direkt nach Verfahren 3 oder $2a_2$ weiter umgesetzt werden.

Verwendet man bei Verfahren 4 3,3-Dimethyl-4-(4'-methoxy-phenacyl)-γ-butyrolacton und Äthanol als Ausgangsstoffe, kann der Reaktionsverlauf durch folgendes Formelschema wiedergegeben werden :

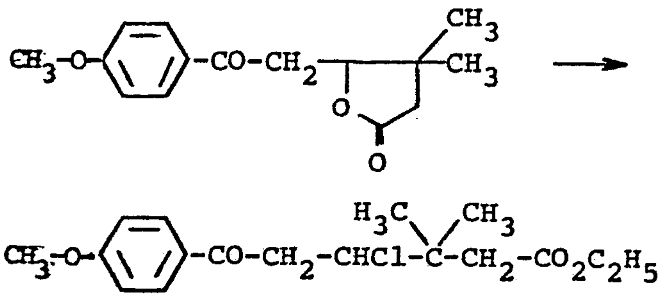

Die beim Verfahren 4 verwendbaren Ausgangsstoffe sind durch die allgemeine Formel (VI) definiert. Die bevorzugten Substituenten $R^1$ und $R^2$ sind die gleichen wie bei Verfahren 3. Die Verbindungen der Formel (VI) sind neu ; ihre Herstellung wird weiter unten beschrieben.

Der zweite Ausgangsstoff für Verfahren 4 ist ein Alkohol der Formel R—OH, wobei die bevorzugte Bedeutung von R die gleiche wie bei Verfahren 3 ist.

Folgende Chlorierungsmittel können bei Verfahren 4 Verwendung finden : Chlorwasserstoff, Thionylchlorid, Phosgen oder Phosphortrichlorid, bevorzugt sind Chlorwasserstoff und Thionylchlorid, gegebenenfalls ist Dimethylformamid als Katalysator.

Grundsätzlich sind Lactonringöffnungen dieser Art bekannt, jedoch enthalten solche Lactone in keinem Fall eine zusätzliche Carbonylgruppe. Es mußten daher Bedingungen gefunden werden, um zu erreichen, daß nur die gewünschte Reaktion abläuft, nicht aber beispielsweise 1) sauer katalysierte Kondensationen des Ketons mit sich selbst (so reagiert beispielsweise Acetophenon unter Chlorwasserstoff Katalyse mit sich selbst) 2) Chlorierung der Carbonylgruppe oder der Wasserstoffatome in α-Position zur Carbonylgruppe.

Erfindungsgemäß wird Verfahren 4 in der Weise durchgeführt, daß der Ausgangsstoff der Formel (VI) in einem Alkohol der Formel R—OH gelöst wird, gegebenenfalls noch ein Verdünnungsmittel zugesetzt wird und anschließend das Chlorierungsmittel eingeleitet oder zugetropft wird. Es setzt eine exotherme Reaktion ein ; um die oben erwähnten Nebenreaktionen zu vermeiden, sollte die Temperatur auf keinen Fall 80 °C überschreiten, vorzugsweise wird jedoch zwischen 20 und 50 °C gearbeitet.

Als Verdünnungsmittel kommen insbesondere Benzol, Toluol, Benzin, Petroläther, Cyclohexan, Chlorbenzol oder Xylol in Frage.

Grundsätzlich ist es auch möglich, zuerst den Lactonring zu öffnen und dann Alkohol zuzusetzen. In diesem Fall erhitzt man die Ausgangsverbindungen (VI) gegebenenfalls in einer der erwähnten Verdünnungsmittel auf Temperaturen zwischen 50 und 80 °C (höhere Temperaturen sind unzweckmäßig) unter Zugabe eines Chlorierungsmittels, gegebenenfalls unter Druck. Anschließend wird der Alkohol R—OH zugetropft oder zugepumpt.

Die Isolierung der Verbindungen der Formel (V) erfolgt durch schonendes Abdestillieren des Lösungsmittels. Eine weitere Reinigung ist schwierig, aber auch nicht notwendig. Die rohen Verbindungen der Formel (V) können direkt für Verfahren 2b verwendet werden.

Verwendet man bei Verfahren 5 3,3-Dimethyl-pent-4-ensäuremethylester und p-Methoxy-benzoylchlorid als Ausgangsstoffe, kann der Reaktionsablauf durch folgendes Formelschema wiedergegeben werden :

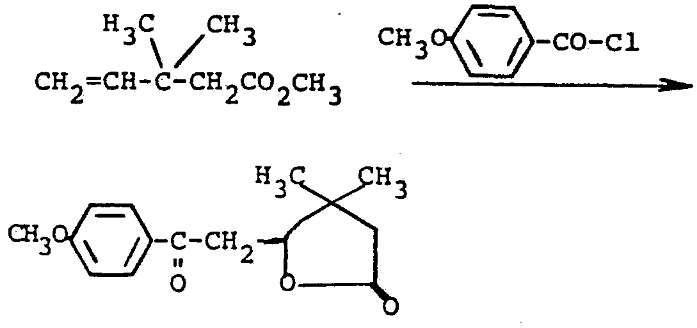

Die beim Verfahren 5 verwendbaren Ausgangsstoffe sind durch die allgemeinen Formeln (VII) und (VIII) definiert. Die bevorzugten Substituenten $R^1$, $R^2$ und $R^3$ sind die gleichen wie bei Verfahren 3. Die Verbindungen der Formel (VII) (DT-OS 2 539 895) und (VIII) sind bekannt.

Beispiele für Verbindungen der Formel (VIII) sind : 4-Methoxybenzoylchlorid, 4-Trifluormethoxybenzoylchlorid, 3-Trifluormethylmercapto-benzoylchlorid, 3-Trifluormethoxy-4-chlorbenzoylchlorid, 3,4-Methylendioxybenzoylchlorid, 3-4-Dimethoxybenzoylchlorid, 4-Trifluormethylmercapto-benzoylchlorid, 4-Ethoxybenzoylchlorid, 3,4-Trifluorethylendioxy-benzoylchlorid.

Als Katalysatoren kommen Friedel-Crafts-Katalysatoren in Frage. Besonders bevorzugt ist Zinntetrachlorid oder gegebenenfalls Mischungen mit Aluminiumchlorid, Titantetrachlorid, Zinkchlorid oder Eisen (III) chlorid. Mit Aluminiumchlorid allein entstehen nicht die Verbindungen der Formel (VI) sondern der Formel (V). Der Friedel-Crafts-Katalysator wird äquimolar im Unterschuß oder im Überschuß eingesetzt. Verfahren 5 kann mit oder ohne Verdünnungsmittel durchgeführt werden. Wird ein solches verwendet, so kommen beispielsweise Methylenchlorid, Chloroform, Dichloräthan, Tetrachloräthan, Nitromethan oder Nitrobenzol in Frage.

Das erfindungsgemäße Verfahren wird in der Weise durchgeführt, daß der Friedel-Crafts-Katalysator, gegebenenfalls in einem Verdünnungsmittel, vorgelegt wird und das Säurehalogenid der allgemeinen Formel (VIII) unter Kühlung zugegeben wird. Anschließend wird ein Ester der allgemeinen Formel (VII) bei Temperaturen zwischen – 25 °C bis + 50 °C, vorzugsweise bei Temperaturen zwischen 0 °C und 25 °C zugetropft.

Es ist jedoch auch möglich, ein Säurehalogenid der Formel (VIII) zusammen mit einem Ester der Formel (VII) gegebenenfalls in Gegenwart eines Verdünnungsmittels, vorzulegen und anschließend den Friedel-Crafts-Katalysator zuzudosieren.

Zur Reaktionsbeschleunigung kann nach beendetem Mischen der Reaktionsteilnehmer bei erhöhter Temperatur gearbeitet werden, z.B. bei 25 bis 150 °C, vorzugsweise bei 30 bis 100 °C. Das Ende der Reaktion ist am Ende der Gasentwicklung zu erkennen. Die Aufarbeitung erfolgt nach dem Sauerstellen in üblicher Weise durch Extraktion. Das rohe Produkt der allgemeinen Formel (VI) kann durch Umkristallisieren gereinigt werden.

Der Reaktionsverlauf ist als äußerst überraschend zu bezeichnen, da eine Lactonbildung unter solch milden Bedingungen nicht bekannt ist.

Verwendet man beim Verfahren 6 zur Herstellung der Verbindungen der Formel (VI) Anisol und 3,3-Dimethyl-4-chlorcarbonylmethyl-γ-butyrolacton als Ausgangsstoffe, kann der Reaktionsablauf durch folgendes Formelschema wiedergegeben werden :

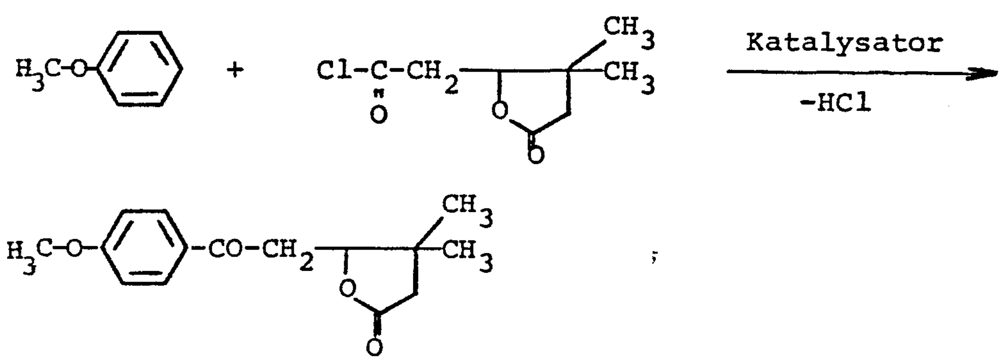


Die beim Verfahren 6 verwendbaren Ausgangsstoffe sind durch die Formeln (X) und (XI) definiert. Die Verbindung (XI) ist neu ; ihre Herstellung wird weiter unten beschrieben.

Die Verbindungen der Formel (X) sind bekannt, beispielsweise seien genannt :

Ethoxybenzol, Methylmercaptobenzol, Anisol, Benzodioxol, Brenzkatechindimethylether. Als Katalysatoren kommen grundsätzlich alle üblichen Friedel-Crafts-Katalysatoren in Frage, wie Aluminiumchlorid, Zinntetrachlorid, Titantetrachlorid, Fluorwasserstoff, Bortrifluorid, Eisen (III)-chlorid, Zinkchlorid, Polyphosphorsäuren, Perfluoralkansulfonsäuren (gegebenenfalls in polymerer Form), sowie gegebenenfalls Mischungen derselben.

Das Verfahren wird bevorzugt in Gegenwart eines Verdünnungsmittels durchgeführt. Es kommen in Frage : Methylenchlorid, Chloroform, Dichloräthan, Tetrachloräthan, Nitrobenzol, Nitromethan. Bevorzugt ist Methylenchlorid.

Die erfindungsgemäße Reaktion ist als äußerst überraschend zu bezeichnen, da erwartet werden mußte, daß auch der Lactonring in folgender Weise reagieren würde :

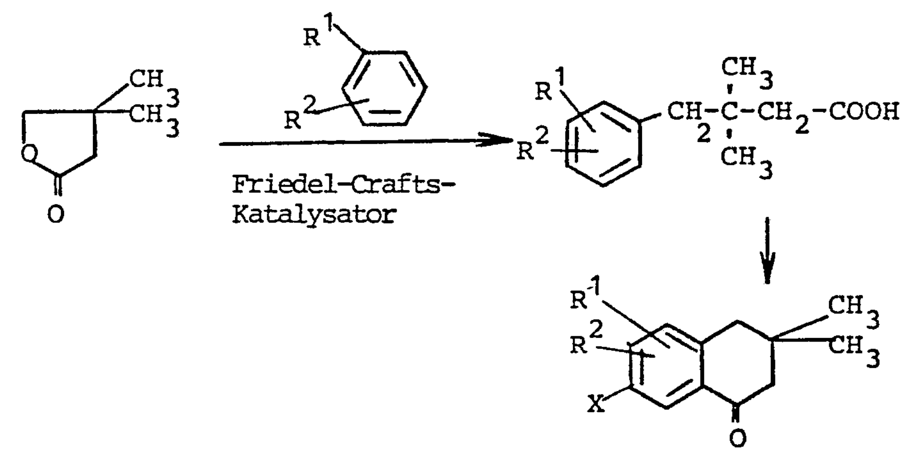


zusätzlich wäre der Ringschluß zum Tetralon zu erwarten gewesen. Ringöffnungen von 5-Ring-Lactonen mit Aromaten nach Friedel-Crafts in der oben formulierten Weise sind bekannt und erfolgen unter sehr milden Bedingungen (Houben-Weyl ; Band VI, 2 Seite 812 ff).

Daß eine solche Reaktion des Lactons mit den Aromaten der Formel (X) nicht erfolgt, ist vor allem auch deshalb überraschend, da der Friedel-Crafts-Katalysator mindestens äquimolar, besser noch im Überschuß, eingesetzt werden muß.

Bevorzugt wird Verfahren 6 wie folgt durchgeführt :

Man legt das Säurechlorid (XI) in einem Verdünnungsmittel vor und fügt bei temperaturen zwischen – 10 und + 5 °C den Friedel-Crafts-Katalysator hinzu. Anschließend tropft man den Aromaten, gegebenenfalls ebenfalls in einem Verdünnungsmittel gelöst, zu. Verwendet man sehr wirksame Friedel-Crafts-Katalysatoren (z.B. Aluminiumchlorid, Zinntetrachlorid) geschieht dies bei – 10 bis + 5 °C, bei weniger wirksamen Friedel-Crafts-Katalysatoren (z.B. Zinkchlorid, Eisenchlorid, Titantetrachlorid, Perfluoralkansulfonsäuren) tropft man den Aromaten bei Raumtemperatur zu. Anschließend rührt man bei Raumtemperatur nach ; bei weniger wirksamen Katalysatoren muß gegebenenfalls bei erhöhter Temperatur gearbeitet werden.

Verwendet man reaktionsträge Aromaten, wie beispielsweise Chlorbenzol, so empfiehlt es sich nicht, die Reaktion durch Temperaturerhöhung zu beschleunigen, sondern die Reaktionszeiten zu verlängern, um die unerwünschte Lactonringöffnung im oben erwähnten Sinn zu verhindern.

Die Aufarbeitung erfolgt in üblicher Weise ; die Lactone können durch Umkristallisieren gereinigt werden.

Verfahren 7 kann durch folgende Reaktionsgleichung beschrieben werden :

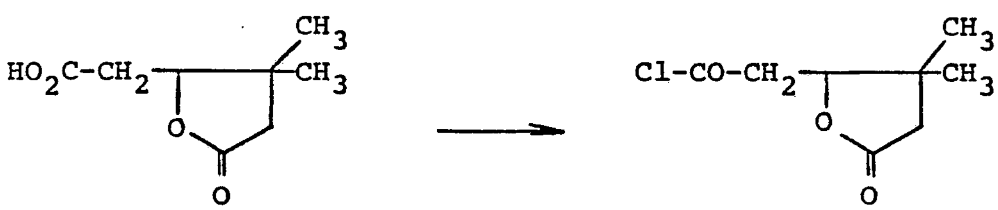

Die als Ausgangsstoff verwendete Säure der Formel (XII) ist bekannt (J. Org. Chem. ; Band 38, Seite 4148 und J. Chem. Soc. 79, 763), nicht jedoch das Säurechlorid der Formel (XI). Daß eine glatte Umwandlung der Säure in das Säurechlorid gelingt, erscheint überraschend, da unter diesen Bedingungen üblicherweise auch eine Ringöffnung des Lactons erfolgt.

Verfahren 7 wird unter den Bedingungen durchgeführt, die zur Herstellung eines Säurechlorids aus einer Säure üblich sind. Bevorzugte Chlorierungsmittel sind Thionylchlorid und Phosgen. Es ist jedoch darauf zu achten, daß möglichst kurze Reaktionszeiten angewendet werden, um eine Nebenreaktion im oben erwähnten Sinne zu vermeiden. Die Aufarbeitung erfolgt in üblicher Weise. Das Säurechlorid kann durch Destillation gereinigt oder roh in Verfahren 6 eingesetzt werden.

Die Styrylcyclopropancarbonsäureester der Formel I (oben) soweit sie mit einem bei Pyrethroiden üblichen Alkohol verestert sind, eignen sich bei guter Pflanzenverträglichkeit und günstiger Warmblütertoxizität zur Bekämpfung von tierischen Schädlingen, insbesondere Insekten, Spinnentieren, die in der Landwirtschaft in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören :

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Phylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage : Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser ; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid ; als feste Trägerstoffe kommen in Frage : z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgier- und/oder schaumerzeugende Mittel kommen in Frage : z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate ; als Dispergiermittel kommen in Frage : z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,000 000 1 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

## Beispiel

Phaedon-Larven-Test

Lösungsmittel : 3 Gewichtsteile Aceton
Emulgator : 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschter Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden ; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik : 17.

## Beispiel 1 (Verfahren 3)

Zu einer Lösung von 34,5 g (0,1 Mol) 6-(4'-Methoxy-phenyl-4,6-dichlor-3,3-dimethyl-hex-5-en-säure-äthylester in 100 ml Äthanol wird bei Raumtemperatur eine Lösung von natriumäthylat (hergestellt aus 2,5 g Natrium und 100 ml Äthanol) getropft. Man rührt 4 h nach, verdünnt mit Eiswasser und stellt neutral. Nach Extrahieren mit Methylenchlorid wird die organische Phase getrocknet und einrotiert. Durch Destillation im Hochvakuum erhält man 26,5 g eines schwach gelblichen Öls vom Siedepunkt 162-168 °C/0,08 mbar. Es enthält alle 4 möglichen Stereoisomeren des 2,2-Dimethyl-3-[2'-chlor-2'-(4'-methoxyphenyl)]-vinyl-cyclopropan-1-carbonsäureäthylesters. Das Vinylproton der Hauptisomeren zeigt (in $CDCl_3$) ein Dublett bei $\delta = 5{,}7$ und 5,85 ppm. Massenspektrum : m/e = 308.

## Beispiel 2 (Verfahren 3)

Eine Lösung von 38,2 g 6-(4'-Methoxy-phenyl)-4,6,6-trichlor-3,3-dimethyl-hexansäureäthylester (roh ; aus Beispiel 8) in 100 ml Äthanol wird bei Raumtemperatur zu einer Lösung von 4,75 g Natrium in 250 ml Äthanol getropft. Man rührt 2 h bei Raumtemperatur nach und erhitzt anschließend noch 1 h auf 50 °C. Dann wird mit Eiswasser versetzt und mit 10 %iger Salzsäure neutralisiert. Nach zweimaligem

Extrahieren mit Methylenchlorid wird die organische Phase getrocknet, einrotiert und im Hochvakuum destilliert. Man erhält 25 g 2,2-Dimethyl-3-[2'-chlor-2'-(4'-methoxyphenyl)] vinyl-cyclopropan-1-carbonsäureäthylester vom Siedepunkt 155-163 °C/0,05 mbar.

Beispiel 3 (Verfahren 3)

Eine Lösung von 35 g 6-(4'-Methoxy-phenyl)-4,6,6-trichlor-3,3-dimethyl-hexansäurechlorid (roh, enthält noch $POCl_3$ ; aus Verfahren 8) in 500 ml Toluol wird unter Eiskühlung mit einer Lösung von 40 g Natrium in 700 ml Äthanol versetzt. Man rührt 8 h bei Raumtemperatur und erhitzt dann noch 1 h auf 50 °C, versetzt mit Eiswasser, neutralisiert mit 10 %iger Salzsäure, trennt die Toluolphase ab und extrahiert die Wasserphase mit Methylenchlorid. Die vereinigten organischen Phasen werden getrocknet und die Lösungsmittel sowie der gebildete Phosphorsäuretriäthylester abdestilliert. Der Rückstand wird durch Destillation im Hochvakuum gereinigt. Man erhält 18,6 g 2,2-Dimethyl-3-[2'-chlor-2'-(4'-methoxy-phenyl)]-vinylcyclopropan-1-carbonsäureäthylester.

Beispiel 4 (Verfahren 3)

Analog Beispiel 3 erhält man aus 6-(4'-Methylenmercaptophenyl)-4,6,6-trichlor-3,3-dimethyl-hexansäurechlorid und Natriumäthylat 2,2-Dimethyl-3-[2'-chlor-2'-(4'-methylmercapto-phenyl)]-vinylcyclopropan-1-carbonsäureäthylester.

Beispiel 5 (Verfahren $2a_1$)

Eine Lösung von 6-(4'-Methoxy-phenyl)-4,6,6-trichlor-3,3-dimethyl-hexansäuremethylester wird in Toluol 1 h auf ca. 40 °C erwärmt. Nach Abdestillieren des Toluols im Vakuum bleibt 6-(4'-Methoxy-phenyl)-4,6-dichlor-3,3-dimethyl-hex-5-en-säuremethylester zurück. Die Struktur wird durch das NMR-Spektrum bewiesen.

Beispiel 6 (Verfahren $2a_2$)

Eine Lösung von 6-(4'-Methoxy-phenyl)-4,6,6-trichlor-3,3-dimethyl-hexancarbonsäurechlorid in Toluol (roh ; enthält noch $POCl_3$ ; aus Verfahren 8) wird mit der 20-fachen äquimolaren Menge trockenen Methanol bei 20 °C versetzt und anschließend noch 1 h auf 40 °C erwärmt. Nach 4 h Nachrühren (ohne Heizung) werden die Lösungsmittel und Phosphorsäureester im Vakuum entfernt. Der Rückstand ist identisch mit dem in Beispiel 5 erhaltenen Produkt.

Beispiel 7 (Verfahren $2a_3$)

Eine Lösung von 26,2 g 3,3-Dimethyl-4-(4'-methoxy-phenacyl)-γ-butyrolacton in 400 ml Toluol wird mit 46 g Phosphorpentachlorid versetzt und solange bei Raumtemperatur gerührt, bis alles $PCl_5$ in Lösung gegangen ist. Anschließend werden bei 20 °C 120 ml Äthanol zugetropft, dann 1 h auf 45 °C erwärmt und noch solange nachgerührt, bis wieder Raumtemperatur erreicht ist. Anchließend wird auf viel Eiswasser gegeben und neutralgestellt. Die Toluolphase wird abgetrennt, getrocknet und einrotiert. Nach Abdestillieren der Lösungsmittel und des Phosphorsäuretriäthylester verbleiben 25 g eines dunklen Öls, das hauptsächlich aus 6-(4'-Methoxy-phenyl)-4,6-dichlor-3,3-dimethyl-hex-5-ensäureäthylester besteht, das nach Verfahren 1a (Beispiel 1) weiterverarbeitet werden kann.

Beispiel 8 (Verfahren 2b)

7,9 g 6-(4'-Methoxy-phenyl)-6-oxo-4-chlor-3,3-dimethyl-hexansäureäthylester werden in 50 ml Toluol gelöst und bei Raumtemperatur 6 g Phosphorpentachlorid zugegeben. Man rührt 9 h bei Raumtemperatur nach, gibt auf 100 ml Eiswasser, stellt neutral und trennt die Toluolphase ab. Nach Trocknen und Abdestillieren des Toluols bei Raumtemperatur bleiben 68 g eines Öls zurück, das Laut NMR-Spektrum aus 6-(4'-Methoxy-phenyl)-4,6,6-trichlor-3,3-dimethyl-hexansäureäthylester besteht. Es kann nach Verfahren 1b weiter umgesetzt werden.

Beispiel 9 (Verfahren 2c)

26,2 g 3,3-Dimethyl-4-(4'-methoxy-phenacyl)-γ-butyrolacton werden in 500 g Toluol gelöst und mit 46 g Phosphorpentachlorid versetzt. Man rührt 18 h bei Raumtemperatur nach und destilliert dann bei Raumtemperatur im Vakuum Phosphoroxychlorid und Toluol ab. Es bleiben 37 g eines braunen Öls zurück, das durch IR und NMR als 6-(4'-Methoxy-phenyl)-4,6,6-trichlor-3,3-dimethyl-hexancarbonsäurechlorid identifiziert wird.

Beispiel 10 (Verfahren 4)

Man löst 78,6 g 3,3-Dimethyl-4-(4'-methoxy-phenacyl)-γ-butyrolacton in 500 ml Äthanol und leitet solange trockenen Chlorwasserstoff ein, bis 50 °C erreicht sind. Dann leitet man bei 50 °C zunächst unter Kühlung 3 h lang einen langsamen Chlorwasserstoffstrom durch die Lösung und leitet anschließend solange weiter ein, bis wieder Raumtemperatur errreicht ist. Durch Abdestillieren des Äthanols im Vakuum erhält man 98 g 6-(4'-Methoxy-phenyl)-6-oxo-4-chlor-3,3-dimethyl-hexansäureäthylester.

Beispiel 11 (Verfahren 4)

Man löst 131 g 3,3-Dimethyl-4-(4'-methoxy-phenacyl)-γ-butyrolacton in 215 g Thionylchlorid in einem 0,7 l Autoklav mit Glaseinsatz. Dann werden 100 g Äthanol zugepumpt und 4 h auf 50 °C gehalten. Nach Abkühlen und Entspannen destilliert man überschüssiges Thionylchlorid bzw. Schwefligsäureäthylester ab. Der Rückstand besteht im wesentlichen aus 6-(4'-Methoxy-phenyl)-6-oxo-4-chlor-3,3-dimethyl-hexansäureäthylester.

Beispiel 12 (Verfahren 4)

Man löst 131 g 3,3-Dimethyl-4-(4'-methoxy-phenacyl)-γ-butyrolacton in 215 g Thionylchlorid in einem 0,7 l Autoklaven mit Glaseinsatz. Dann werden 100 g Methanol zugepumpt und 4 h auf 50 °C erhitzt. Nach Aufarbeitung wie in Beispiel 10 erhält man 6-(4'-Methoxy-phenyl)-6-oxo-4-chlor-3,3-dimethyl-hexansäuremethylester.

Beispiel 13 (Verfahren 5)

Zu der Mischung aus 85,5 g (0,5 mol) p-Methoxybenzoylchlorid und 130,3 g (0,5 mol) Zinntetrachlorid wurden 71 g (0,5 mol) 3,3-Dimethyl-4-pentensäuremethylester bei 20 °C unter Eiskühlung getropft. Nach dem Stehen über Nacht war die Reaktionsmischung erstarrt. Sie wurde in Methylenchlorid aufgenommen, filtriert und mit verd. Salzäure ausgeschüttelt. Nacht Trocknen und Anziehen des Lösungsmittels im Vakuum erhält man ein Rohprodukt, das durchkristallisierte. Das Umkristallisieren aus Ethanol liefert 3,3-Dimethyl-4-(4'-methoxy-phenacyl)-γ-butyrolacton vom Fp. 132-134 °C.

Beispiel 14 (Verfahren 5)

In eine Lösung von 17,1 g (0,1 mol) p-Methoxybenzoylchlorid und 14,2 g (0,1 mol) 3,3-Dimethyl-4-pentensäuremethylester in 100 ml Dichlormethan wurden bei 0° 26 g (0,1 mol) Zinntetrachlorid getropft und anschließend 8 Stunden bis zum Ende der Gasentwicklung unter Rückfluß erhitzt. Nach der Aufarbeitung mit verdünnter Salzäure erhielt man 23,9 g (91 %) 3,3-Dimethyl-4-(4'-methoxyphenacyl)-γ-butyrolacton vom Fp. 132-134 °C (aus Ethanol).

Beispiel 15 (Verfahren 7)

Man vermischt 17,2 g 3,3-Dimethyl-4-carboxymethyl-γ-butyrolacton mit 60 ml Thionylchlorid und erhitzt 1 h auf 80 °C. Dann destilliert man das überschüssige Thionylchlorid bei Normaldruck ab, die letzten Reste im Wasserstrahlvakuum. Der Rückstand besteht aus 3,3-Dimethyl-4-chlorcarbonylmethyl-γ-butyrolacton und kann direkt für Verfahren 13 verwendet werden. Es kann jedoch auch noch durch Destillation weiter gereinigt werden : Siedepunkt : 130-140 °C/0,3 mbar.

Beispiel 16 (Verfahren 6)

Man legt 80 g Aluminiumchlorid in 300 ml Methylenchlorid vor und tropft 59 g 3,3-Dimethyl-4-chlorcarbonylmethyl-γ-butyrolacton in 150 ml Methylenchlorid gelöst bei 0-5 °C zu. Anschließend tropft man 32,4 g Anisol in 50 ml Methylenchlorid gelöst ebenfalls bei 0-5 °C zu. Dann laßt man auf Raumtemperatur kommen und rührt noch 7 h bei Raumtemperatur nach. Nach Eingießben des Ansatzes in Eiswasser wird die organische Phase abgetrennt und neutral gewaschen. Nach Trocknen und Abdestillieren des Lösungsmittels erhält man rohes 3,3-Dimethyl-4-(4'-methoxy-phenacyl)-γ-butyrolacton, das aus Äthanol umkristallisiert wird. Schmp. 132-134 °C.

Beispiel 17

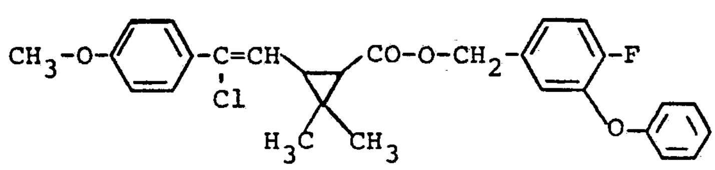

4,4 g (0,02 Mol) 3-Phenoxy-4-fluoro-benzylalkohol und 7,1 g (0,02 Mol) 2,2-Dimethyl-3-(2-chloro-2(4-methoxy-phenyl)-vinyl)-cyclopropancarbonsäurechlorid werden in 100 ml wasserfreiem Toluol gelöst und bei 20-25 °C 2,5 g Pyridin, gelöst in 50 ml wasserfreiem Toluol, unter Rühren zugetropft. Anschließend wird weitere 3 Stunden bei 25-35 °C gerührt. Das Reaktionsgemisch wird in 150 ml Wasser, dem 10 ml konzentrierte Salzsäure zugesetzt werden, gegossen, die organische Phase abgetrennt und nochmals mit 100 ml Wasser gewaschen. Anschließend wird die Toluolphase über Natriumsulfat getrocknet und das Lösungsmittel im Wasserstrahlvakuum abdestilliert. Letzte Lösungsmittelreste werden durch kurzes Andestillieren bei 60 °C/l Torr Badtemperatur entfernt. Man erhält 8,1 g (84,5 % der Theorie) 2,2-Dimethyl-3-(2-chloro-2-(4-trifluormethoxyphenyl)-vinyl)-cyclopropancarbonsäure-(4-fluoro-3-phenoxy-benzyl)-ester.

Analog wird erhalten :

18) 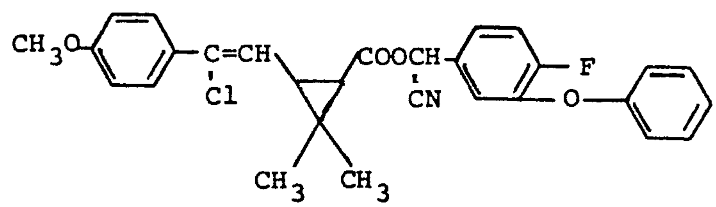

## Ansprüche

1. Verbindungen der Formel

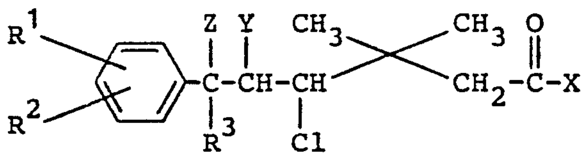

in welcher

X für Cl oder —O—R steht,

R für $C_{1-4}$-Alkyl oder für den Rest eines gegebenenfalls substituierten Phenoxybenzylalkohols der Formel

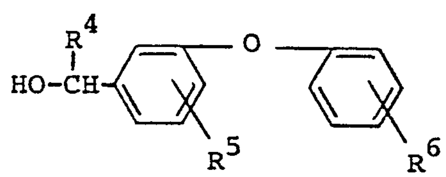

in welcher

$R^4$ für Wasserstoff, CN, —C ≡ CH steht,

$R^5$ und $R^6$ für Wasserstoff oder Fluor stehen,

Y für Wasserstoff steht

Z für Chlor oder gemeinsam mit Y für eine Doppelbindung steht,

$R^1$ für Alkoxy oder Alkylthio, die gegebenenfalls durch Halogen substituiert sind, steht,

$R^2$ für Wasserstoff, Alkoxy oder gemeinsam mit $R^1$ für gegebenenfalls halogensubstituiertes Alkylendioxy steht,

$R^3$ für Wasserstoff oder Chlor steht.

2. Verfahren zur Herstellung von Verbindungen der Formel

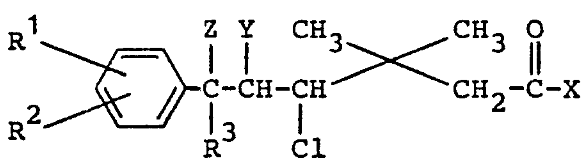

in welcher

X für Cl oder —O—R steht,

R für $C_{1-4}$-Alkyl oder für den Rest eines gegebenenfalls substituierten Phenoxybenzylalkohols der Formel

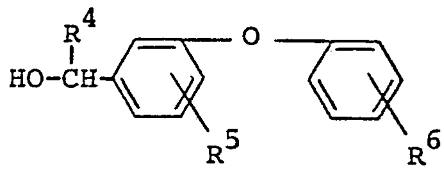

in welcher
$R^4$ für Wasserstoff, CN, —C ≡ CH steht,
$R^5$ und $R^6$ für Wasserstoff oder Fluor stehen,
Y für Wasserstoff steht
Z für Chlor oder gemeinsam mit Y für eine Doppelbindung steht,
$R^1$ für Alkoxy oder Alkylthio, die gegebenenfalls durch Halogen substituiert sind, steht,
$R^2$ für Wasserstoff, Alkoxy oder gemeinsam mit $R^1$ für gegebenenfalls halogensubstituiertes Alkylendioxy steht,
$R^3$ für Wasserstoff oder Chlor steht,
dadurch gekennzeichnet, daß man
a) für den Fall, daß Y und Z gemeinsam für eine Doppelbindung stehen,
$a_1$) aus Verbindungen der obigen Formel,
in welcher
Z und $R^3$ für Chlor stehen und
Y für Wasserstoff steht, thermisch Chlorwasserstoff abspaltet, oder
$a_2$) Verbindungen der obigen Formel,
in welcher
Z und $R^3$ für Chlor stehen und
Y für Wasserstoff steht und
X für Chlor steht, mit Alkoholen der Formel

R—O—H

in welcher
R die oben angegebene Bedeutung besitzt, umsetzt, oder
$a_3$) Verbindungen der Formel VI

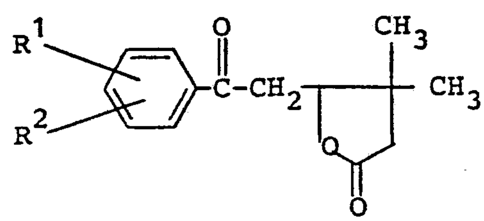 (VI)

in welcher
$R^1$ und $R^2$ die oben angegebene Bedeutung haben, mit mindestens zwei Äquivalenten Phosphorpentachlorid umsetzt und anschließend mit einem Alkohol der Formel

R—O—H

in welcher
R die oben angegebene Bedeutung besitzt, umsetzt oder
$a_4$) Verbindungen der Formel (VI) (oben) reduziert, Wasser abspaltet und mit einem Chlorierungsmittel in Gegenwart eines Alkohols der Formel

R—OH

in welcher
R die oben angegebene Bedeutung besitzt, umsetzt oder aber nacheinander zunächst den Lactonring mit einem Chlorierungsmittel öffnet und anschließend mit dem Alkohol umsetzt oder
b) für den Fall, daß Z und $R^3$ für Cl stehen und Y für H steht, Verbindungen der Formel (V)

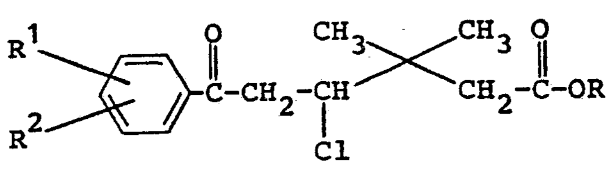 (V)

in welcher

R, $R^1$, $R^2$ die oben angegebene Bedeutung besitzen, mit Phosphorpentachlorid in einem Verdünnungsmittel unterhalb 30 °C umsetzt, oder

c) für den Fall, daß Z und $R^3$ für Cl stehen und Y für H steht und X für Cl steht, Verbindungen der Formel (VI) (oben)

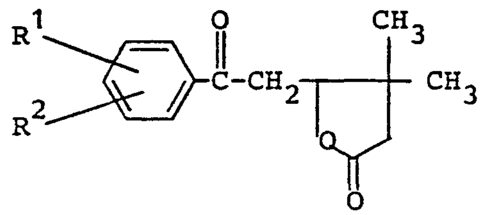

(VI)

in welcher

$R^1$ und $R^2$ die oben angegebene Bedeutung haben, mit mindestens zwei Äquivalenten Phosphorpentachlorid in einem Verdünnungsmittel unterhalb 30 °C umsetzt.

3. Verwendung von Verbindungen der Formel

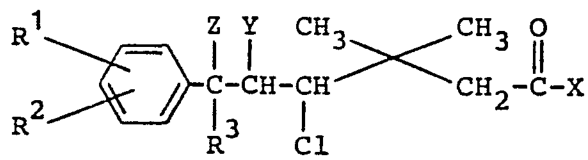

in welcher

X für Cl oder —O—R steht,

R für $C_{1-4}$-Alkyl oder für den Rest eines gegebenenfalls substituierten Phenoxybenzylalkohols der Formel

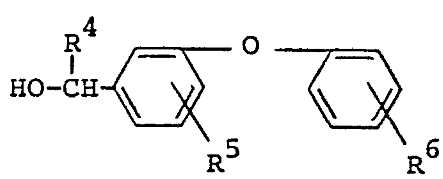

in welcher

$R^4$ für Wasserstoff, CN, —C ≡ CH steht,

$R^5$ und $R^6$ für Wasserstoff oder Fluor stehen,

Y für Wasserstoff steht

Z für Chlor oder gemeinsam mit Y für eine Doppelbindung steht,

$R^1$ für Alkoxy oder Alkylthio, die gegebenenfalls durch Halogen substituiert sind, steht,

$R^2$ für Wasserstoff, Alkoxy oder gemeinsam mit $R^1$ für gegebenenfalls halogensubstituiertes Alkylendioxy steht,

$R^3$ für Wasserstoff oder Chlor steht, zur Herstellung von Styryl-cyclopropancarbonsäureestern der allgemeinen Formel (I)

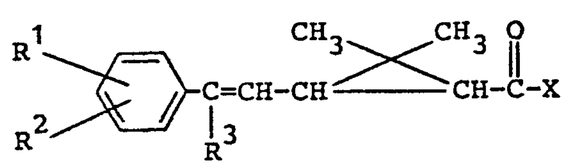

(I)

in der

X, $R^1$, $R^2$, $R^3$ die oben angegebene Bedeutung haben, dadurch gekennzeichnet, daß man Verbindungen der Formel

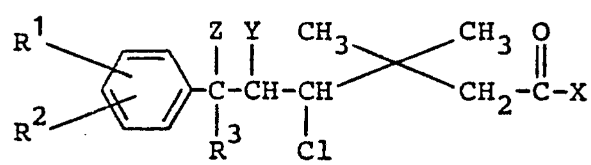

in welcher

$R^1$, $R^2$, $R^3$, X, Y, Z die oben angegebene Bedeutung haben, mit einer äquivalenten Menge einer Base gegebenenfalls in Gegenwart eines Verdünnungsmittels zwischen − 20 und 60 °C umsetzt, und gegebenenfalls mit einem Alkohol der Formel R—O—H weiter umsetzt.

4. Verbindungen der Formel (II)

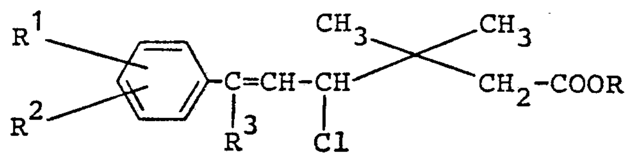

(II)

in welcher

R-$R^3$ die in Anspruch 1 angegebene Bedeutung haben.

5. Verbindungen der Formel (III)

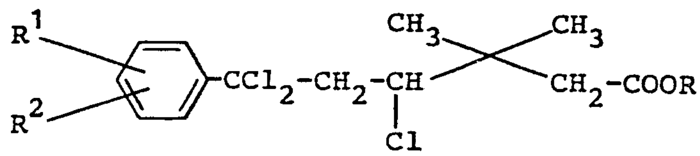

(III)

in welcher

R-$R^3$ die in Anspruch 1 angegebene Bedeutung haben.

6. Verbindungen der Formel (IV)

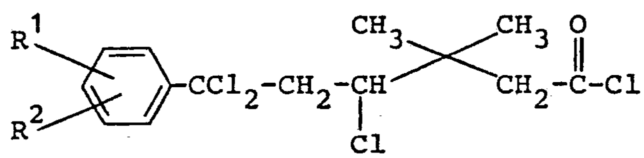

(IV)

in welcher

$R^1$ und $R^2$ die in Anspruch 1 angegebene Bedeutung haben.

**Claims**

1. Compounds of the formula

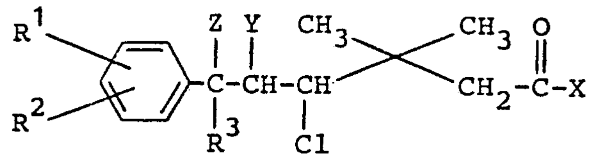

in which

X represents Cl or —O—R,

R represents $C_{1-4}$-alkyl or the radical of an optionally substituted phenoxybenzyl alcohol of the formula

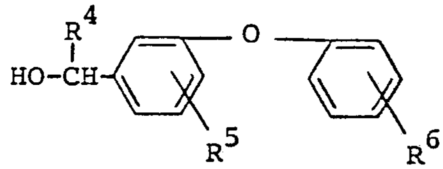

in which

$R^4$ represents hydrogen, CN or —C ≡ CH, and

$R^5$ and $R^6$ represent hydrogen or fluorine,

Y represents hydrogen,

Z represents chlorine or together with Y represents a double bond,

$R^1$ represents alkoxy or alkylthio, which are optionally substituted by halogen,
$R^2$ represents hydrogen or alkoxy or together with
$R^1$ represents optionally halogen-substituted alkylenedioxy, and
$R^3$ represents hydrogen or chlorine.

Process for the preparation of compounds of the formula

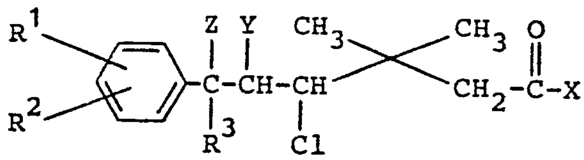

in which
X represents Cl or —O—R,
R represents $C_{1-4}$-alkyl or the radical of an optionally substituted phenoxybenzyl alcohol of the formula

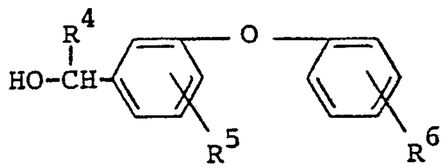

in which
$R^4$ represents hydrogen, CN or —C ≡ CH, and
$R^5$ and $R^6$ represent hydrogen or fluorine,
Y represents hydrogen,
Z represents chlorine or together with Y represents a double bond,
$R^1$ represents alkoxy or alkylthio, which are optionally substituted by halogen,
$R^2$ represents hydrogen or alkoxy or together with
$R^1$ represents optionally halogen-substituted alkylenedioxy, and
$R^3$ represents hydrogen or chlorine,
characterised in that
a) in the case where Y and Z together represent a double bond
$a_1$) hydrogen chloride is split off, by the action of heat, from compounds of the above formula in which
Z and $R^3$ represent chlorine and
Y represents hydrogen, or
$a_2$) compounds of the above formula
in which
Z and $R^3$ represent chlorine and
Y represents hydrogen and
X represents chlorine, are reacted with alcohols of the formula

R—O—H

in which
R has the meaning indicated above, or
$a_3$) compounds of the formula VI

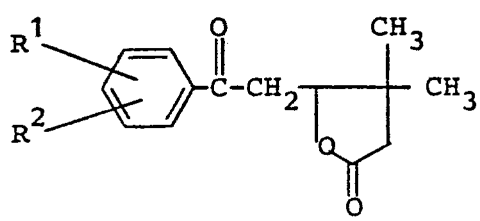

(VI)

in which
$R^1$ and $R^2$ have the meaning indicated above, are reacted with at least two equivalents of phosphorus pentachloride and the resulting reaction solution is then reacted with an alcohol of the formula

R—O—H

in which

R has the meaning indicated above, or

$a_4$) compounds of the formula (VI) (above) are reduced, water is split off from the compounds obtained and the products obtained are reacted with a chlorinating agent in the presence of an alcohol of the formula

R—OH

in which

R has the meaning indicated above, or, successively, the lactone ring is first opened with a chlorinating agent and the product is then reacted with the alcohol, or

b) in the case where Z and $R^3$ represent Cl and Y represents H, compounds of formula (V)

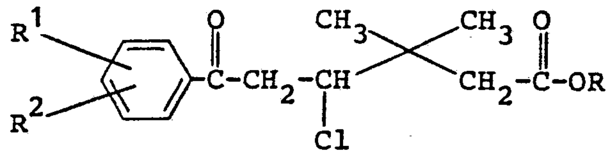

(V)

in which

R, $R^1$ and $R^2$ have the meaning indicated above, are reacted with phosphorous pentachloride in a diluent below 30 °C, or

c) in the case where Z and $R^3$ represent Cl and Y represents H and X represents Cl, compounds of the formula (VI) (above)

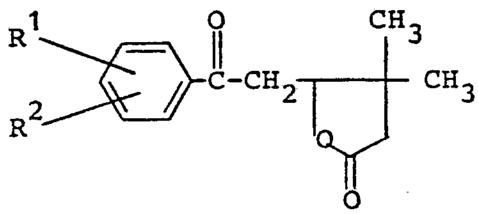

(VI)

in which

$R^1$ and $R^2$ have the meaning indicated above, are reacted with at least two equivalents of phosphorus pentachloride in a diluent below 30 °C.

3. Use of compounds of the formula

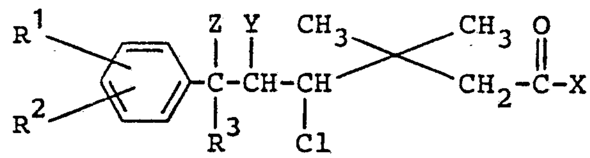

in which

X represents Cl or —O—R,

R represents $C_{1-4}$-alkyl or the radical of an optionally substituted phenoxybenzyl alcohol of the formula

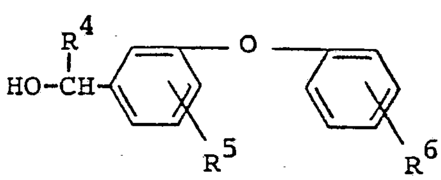

in which

$R^4$ represents hydrogen, CN or —C ≡ CH, and

$R^5$ and $R^6$ represent hydrogen or fluorine,

Y represents hydrogen,

Z represents chlorine or together with Y represents a double bond,

$R^1$ represents alkoxy or alkylthio, which are optionally substituted by halogen,

$R^2$ represents hydrogen or alkoxy or together with

$R^1$ represents optionally halogen-substituted alkylenedioxy, and

$R^3$ represents hydrogen or chlorine, for the preparation of styryl-cyclopropanecarboxylic acid esters of the general formula (I)

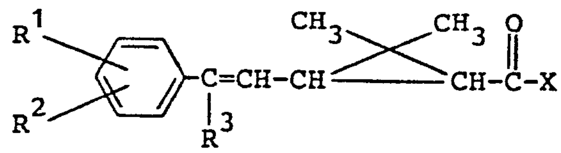

(I)

in which

X, $R^1$, $R^2$ and $R^3$ have the meaning indicated above, characterised in that compounds of the formula

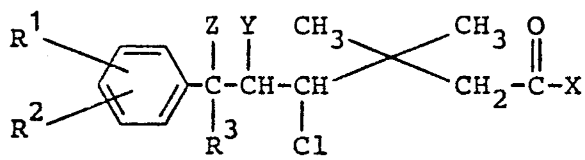

in which

$R^1$, $R^2$, $R^3$, X, Y and Z have the meaning indicated above, are reacted with an equivalent amount of a base, if appropriate in the presence of a diluent, between − 20 and 60 °C, and if appropriate the product is further reacted with an alcohol of the formula R—O—H.

4. Compounds of the formula (II)

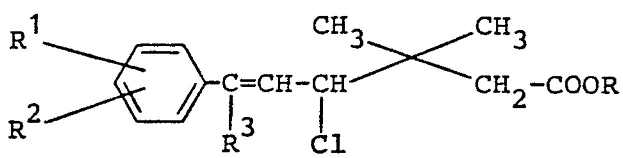

(II)

in which

R-$R^3$ have the meaning indicated in Claim 1.

5. Compounds of the formula (III)

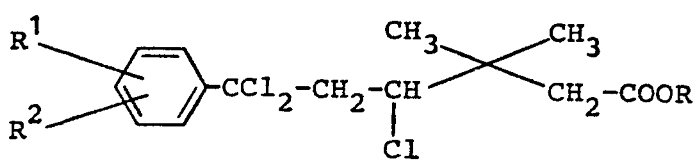

(III)

in which

R-$R^3$ have the meaning given in Claim 1.

6. Compounds of the formula (IV)

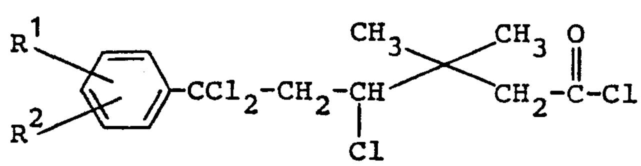

(IV)

in which

$R^1$ and $R^2$ have the meaning indicated in Claim 1.

**Revendications**

1. Composés de formule :

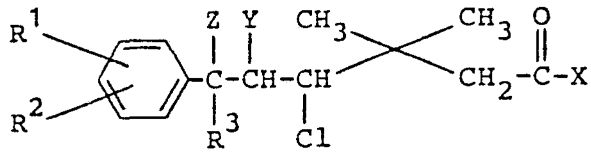

dans laquelle

X représente Cl ou —O—R,

R est un groupe alkyle en $C_1$ à $C_4$ ou le reste d'un alcool phénoxybenzylique éventuellement substitué de formule :

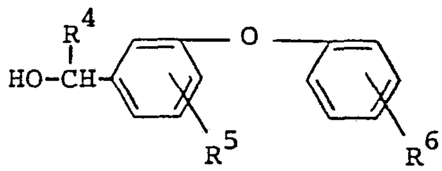

dans laquelle

$R^4$ représente l'hydrogène, CN, —C ≡ CH,

$R^5$ et $R^6$ représente de l'hydrogène ou du fluor,

Y est de l'hydrogène,

Z est du chlore ou forme conjointement avec Y une double liaison,

$R^1$ est un groupe alkoxy ou un groupe alkylthio qui sont éventuellement substitués par un halogène,

$R^2$ est de l'hydrogène, un groupe alkoxy ou forme, conjointement avec $R^1$, un groupe alkylènedioxy éventuellement substitué par un halogène,

$R^3$ est de l'hydrogène ou du chlore.

2. Procédé de production de composés de formule :

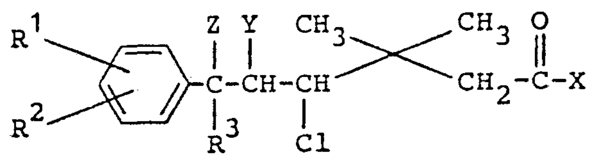

dans laquelle

X représente Cl ou —O—R,

R est un groupe alkyle en $C_1$ à $C_4$ ou le reste d'un alcool phénoxybenzylique éventuellement substitué de formule :

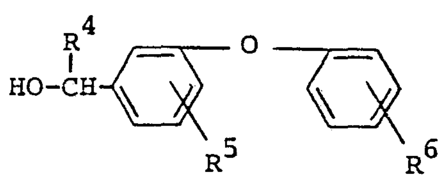

dans laquelle

$R^4$ représente de l'hydrogène, CN, —C ≡ CH,

$R^5$ et $R^6$ représentent de l'hydrogène ou du fluor,

Y est de l'hydrogène,

Z est du chlore ou forme conjointement avec Y une double liaison,

$R^1$ est un groupe alkoxy ou un groupe alkylthio qui sont éventuellement substitués par un halogène,

$R^2$ représente de l'hydrogène, un groupe alkoxy ou forme conjointement avec $R^1$ un groupe alkylènedioxy éventuellement substitué par un halogène, est de l'hydrogène ou du chlore, caractérisé en ce que

a) au cas où Y et Z forment en commun une double liaison,

$a_1$) on élimine thermiquement du chlorure d'hydrogène de composés de la formule ci-dessus, dans laquelle

Z et $R^3$ représentent du chlore et

Y est de l'hydrogène, ou

$a_2$) on fait réagir des composés de la formule ci-dessus, dans laquelle

Z et $R^3$ représentent du chlore et

Y est de l'hydrogène et
X est du chlore, avec des alcools de formule

R—O—H

dans laquelle
R a la définition indiquée ci-dessus, ou
$a_3$) on fait réagir des composés de formule VI :

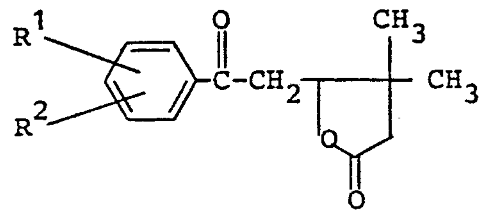

(VI)

dans laquelle
$R^1$ et $R^2$ ont la définition indiquée ci-dessus, avec au moins deux équivalents de pentachlorure de phosphore, puis avec un alcool de formule

R—O—H

dans laquelle
R a la définition indiquée ci-dessus, ou
$a_4$) on réduit des composés de formule (VI) (ci-dessus), on élimine de l'eau et on les fait réagir avec un agent de chloration en présence d'un alcool de formule

R—O—H

dans laquelle
R a la définition indiquée ci-dessus, ou bien, successivement, on ouvre d'abord le noyau de lactone avec un agent de chloration, puis on les fait réagir avec un alcool, ou
b) au cas où Z et $R^3$ représentent Cl et Y représente H, on fait réagir des composés de formule (V) :

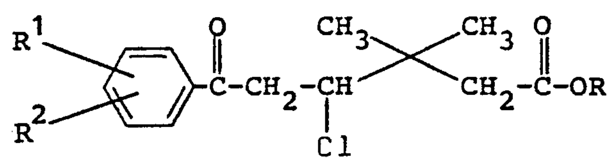

(V)

dans laquelle
R, $R^1$, $R^2$ ont la définition indiquée ci-dessus, avec du pentachlorure de phosphore dans un diluant au-dessous de 30 °C, ou
c) au cas où Z et $R^3$ représentent Cl et Y représente H et X représente Cl, on fait réagir des composés de formule (VI) (ci-dessus)

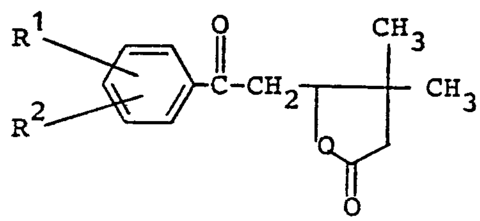

(VI)

dans laquelle
$R^1$ et $R^2$ ont la définition indiquée ci-dessus, avec au moins deux équivalents de pentachlorure de phosphore dans un diluant, au-dessous de 30 °C.
3. Utilisation de composés de formule :

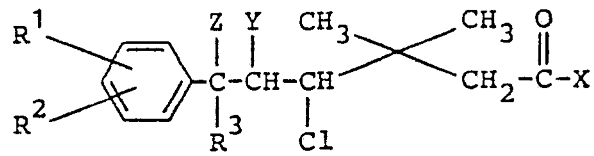

dans laquelle

X représente Cl ou —O—R,

R est un groupe alkyle en $C_1$ à $C_4$ ou le reste d'un alcool phénoxybenzylique éventuellement substitué, de formule :

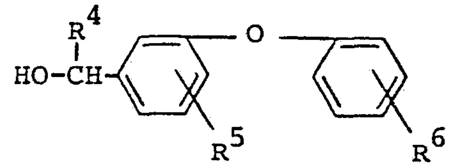

dans laquelle

$R^4$ représente l'hydrogène, CN, —C ≡ CH,

$R^5$ et $R^6$ représentent de l'hydrogène ou du fluor,

Y est de l'hydrogène,

Z est du chlore ou forme conjointement avec Y une double liaison,

$R^1$ est un groupe alkoxy ou un groupe alkylthio qui sont éventuellement substitués par un halogène,

$R^2$ est de l'hydrogène, un groupe alkoxy ou forme conjointement avec $R^1$ un groupe alkylènedioxy éventuellement substitué par un halogène,

$R^3$ est de l'hydrogène ou du chlore, pour la production d'esters d'acides styryl-cyclopropane-carboxyliques de formule générale (I) :

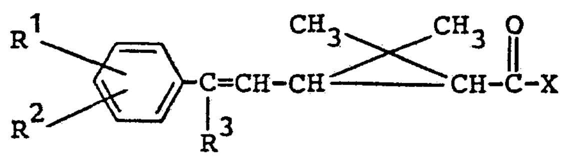

(I)

dans laquelle

X, $R^1$, $R^2$, $R^3$ ont la définition indiquée ci-dessus, caractérisé en ce qu'on fait réagir des composés de formule :

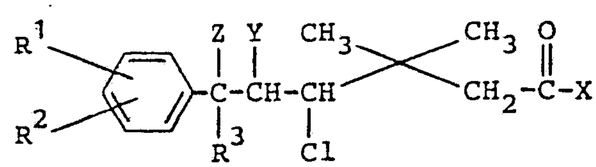

dans laquelle

$R^1$, $R^2$, $R^3$, X, Y, Z ont la définition indiquée ci-dessus, avec une quantité équivalente d'une base, éventuellement en présence d'un diluant, entre − 20 et 60 °C, et on poursuit éventuellement la réaction avec un alcool de formule R—O—H.

4. Composés de formule (II) :

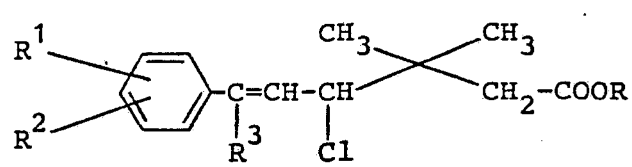

(II)

dans laquelle

R-$R^3$ ont la définition indiquée dans la revendication 1.

5. Composés de formule (III) :

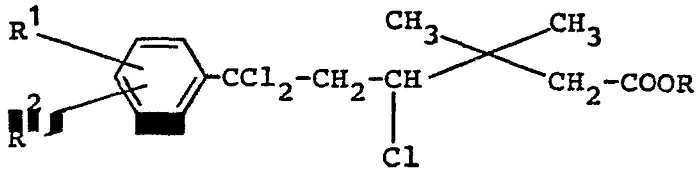

(III)

dans laquelle

R-$R^3$ ont la définition indiquée dans la revendication 1.

6. Composés de formule (IV) :

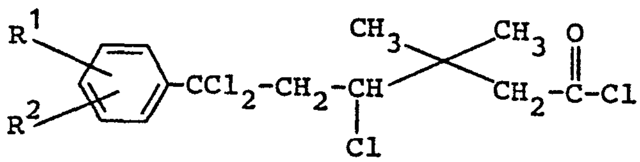

(IV)

dans laquelle

$R^1$ et $R^2$ ont la définition indiquée dans la revendication 1.